# EUROPÄISCHE PATENTSCHRIFT

(11) **EP 1 242 101 B1**
(45) Veröffentlichungstag und Bekanntmachung des Hinweises auf die Patenterteilung: **21.06.2006**
(21) Anmeldenummer: 00991583.6
(22) Anmeldetag: 05.12.2000
(51) Int. Cl.: A61K 35/32, A61L 27/22

(54) **VERFAHREN ZUR HERSTELLUNG EINES MIT KNOCHENWACHSTUMSFAKTOREN ANGEREICHERTEN KNOCHENMATERIALS**
METHOD FOR PRODUCING A BONE MATERIAL ENRICHED WITH BONE GROWTH FACTORS
PROCEDE DE PRODUCTION D'UN MATERIAU OSSEUX ENRICHI EN FACTEURS DE REGULATION DE LA CROISSANCE OSSEUSE

(30) Priorität: 22.12.1999 DE 19962248
(43) Veröffentlichungstag der Anmeldung: 25.09.2002
(73) Patentinhaber: Tutogen Medical GmbH, 91077 Neunkirchen am Brand (DE)
(72) Erfinder: BAUMGARTNER, Ludwig, 90425 Nürnberg (DE)
(74) Vertreter: Manitz, Finsterwald & Partner GbR
(86) Internationale Anmeldenummer: PCT/EP2000/012234
(87) Internationale Veröffentlichungsnummer: WO 2001/045720

(56) Entgegenhaltungen:
- WO-A-96/39203
- WO-A-97/31661
- WO-A-98/40113
- GB-A- 2 164 042
- US-A- 4 394 370
- US-A- 4 563 489
- US-A- 4 975 527

## Beschreibung

Für das Wachstum von menschlichen oder tierischen Knochen spielen Wachstumsfaktoren, sogenannte BMP's (Bone Morphogenetic Protein), eine entscheidende Rolle. Zur Gewinnung dieser Wachstumsfaktoren wurden zweckmäßige Verfahren entwickelt, bei denen Knochenmaterial demineralisiert und Wachstumsfaktoren in dem demineralisierten Knochenmaterial angereichert wurden. Das mit Wachstumsfaktoren angereicherte Knochenmaterial wird insbesondere in der Chirurgie eingesetzt, um das Knochenwachstum an Defektstellen zu fördern und den Heilungsprozeß des Knochens zu beschleunigen.

Weiterhin sind Verfahren zur Trennung und Gewinnung unterschiedlicher Wachstumsfaktoren aus Knochen bekannt. Abweichend davon können präparative Mengen von Knochenwachstumsfaktoren auf gentechnischem Wege gewonnen werden.

Bei bisher praktizierten Verfahren ist es nachteilig, daß bei einer Verwendung eines mit Knochenwachstumsfaktoren angereicherten Knochenmaterials in der Chirurgie die Knochenwachstumsfaktoren vorzeitig ausgeschwemmt werden, so daß sich das beschleunigte Knochenwachstum nicht über den gesamten Heilungsprozeß des Knochens aufrecht erhalten läßt.

Aus der WO-A-97/31661 ist ein Verfahren zur Herstellung von mit BMP beladenem Knochenmaterial bekannt, bei dem ein Träger, z.B. demineralisierter, kortikaler Knochen, in eine BMP-Kollagenlösung unter Anlegung eines Vakuums eingetaucht wird und in einem weiteren Verfahrensschritt lyophilisiert wird.

Aus der WO-A-98/40113 ist die Herstellung eines Knochenmaterials bekannt, wobei demineralisiertes Knochenpulver optional unter Zugabe von BMP in eine Gelatinematrix eingebettet wird.

Aus der US-A-4,394,370 ist ein Verfahren zur Herstellung eines Knochenmaterials bekannt, bei dem DBP und optional BMP in eine Kollagenmatrix unter Lyophilisieren eingebaut werden.

Aus der WO-A-96/39203 ist ein Verfahren zur Herstellung von Knochenmaterial bekannt, bei dem DBM zu einer Matrix bestehend aus Kollagen und BMP gegeben wird. Das Material wird anschließend lyophilisiert.

Aus der US-A-4,563,489 ist es bekannt, BMP in Chloroform-Lösung mit Polymer-Kunststoffen zu mischen und daraus Formkörper herzustellen, die direkt in lebendes Gewebe implantiert werden.

Der Erfindung liegt die Aufgabe zugrunde, ein Verfahren bereitzustellen, mit dem ein mit Knochenwachstumsfaktoren angereichertes Knochenmaterial mit verbesserter Freisetzungsrate hergestellt werden kann.

Diese Aufgabe wird erfindungsgemäß durch ein Verfahren mit den Merkmalen des Anspruchs 1 gelöst.

Die Erfindung basiert auf der Erkenntnis, daß auch die Oberfläche und/oder das Innere von Knochenmaterial mit Hilfe eines geeigneten Verfahrens von einem resorbierbaren Stoff ummantelt werden kann, wobei die Ummantelung eines mit Knochenwachstumsfaktoren angereicherten oder beladenen Knochenmaterials dann die beabsichtigte verzögerte Abgabe der Knochenwachstumsfaktoren bewirkt. Hierdurch können bei einer chirurgischen Anwendung des Knochenmaterials als Transplantat die Knochenwachstumsfaktoren kontinuierlich über längere Zeit und über den gesamten Heilungsprozeß eines Knochens abgegeben werden, wobei die Dauer und Geschwindigkeit der Abgabe von den Eigenschaften der Ummantelung abhängig ist. Eine Steuerung der Wirkstoffabgabe ist beispielsweise über die Dicke und die stoffliche Zusammensetzung der Ummantelung möglich.

Vorteilhafte Ausführungsformen der Erfindung sind in der Beschreibung und den Unteransprüchen beschrieben.

Vorteilhafterweise wird eine Lösung oder Suspension der Knochenwachstumsfaktoren auf die Oberfläche eines geeigneten Knochenmaterials, beispielsweise bovine Spongiosa, das auf herkömmliche Weise von Knochenmark befreit wurde, aufgesprüht oder aufgeträufelt Ferner ist es möglich, das Knochenmaterial in die Lösung einzutauchen, so daß die Lösung von der Oberfläche des Knochenmaterials eingesaugt: wird. Hierbei werden die Knochenwachstumsfaktoren überwiegend, jedoch nicht ausschließlich in einem Oberflächenbereich des Knochenmaterials angelagert, wobei im Anschluß an diese Behandlung das mit Knochenwachstumsfaktoren angereicherte Knochenmaterial ummantelt wird.

Um die Knochenwachstumsfaktoren vermehrt auch im Inneren des Knochenmaterials anzureichern, wird in einer Weiterentwicklung beim Aufsprühen, Aufträufeln oder Einsaugen der Lösung oder Suspension ein Vakuum angelegt. Durch das angelegte Vakuum wird Luft aus dem Inneren des Knochenmaterials abgesaugt, die sonst das Eintreten von Lösungsmittel oder Suspension verhindert.

Bei einer weiteren Ausführungsvariante wird das beladene und somit mit Knochenwachstumsfaktoren angereicherte Knochenmaterial zur Ummäntelung in vorgefertigte Kapseln, Mulden oder Taschen aus resorbierbarem Stoff eingebettet.

Vorteilhafterweise wird als resorbierbarer Stoff Kollagen verwendet, das gelöst oder dispergiert ist oder als Gel vorliegt. Weitere Beispiele für resorbierbare Stoffe sind Gelatine oder Oxycellulose. Das Knochenmaterial kann in Form von Blöcken, Chips oder Pulver eingesetzt werden. Es kann erimdungsgemäß in üblicher Mineral/ Matrix-Zusammensetzung vorliegen. Zweckmäßigerweise wird jedoch in dem erfindungsgemäßen Verfahren Knochenmaterial eingesetzt, das ganz oder teilweise demineralisiert ist. Das eingesetzte Knochenmaterial kann sowohl tierischen als auch menschlichen Ursprungs sein.

Bei einer zweckmäßigen Weiterentwicklung wird das Knochenmaterial auch mit zumindest einem Antibiotikum beladen. Die Beladung kann auf beliebige Art und Weise beispielsweise durch Zugabe des Antibiotikums zur Lösung oder Suspension des resorbierbaren Stoffs oder durch Aufträufeln des Antibiotikums auf das Knochenmaterial erfolgen. Vorzugsweise wird das beladene und mit resorbierbarem Stoff ummantelte Knochenmaterial gefriergetrocknet oder durch Wärme getrocknet.

In einer weiteren zweckmäßigen Weiterentwicklung wird das beladene und ummantelte Knochenmaterial keimfrei hergestellt oder nach dem Trocknen sterilisiert.

Nachfolgend werden zwei Ausführungsbeispiele beschrieben, die nicht unter das beanspruchte Verfahren fallen:

### Beispiel 1

100 ml einer in bekannter Weise hergestellten 0,5%igen Kollagenlösung werden in einem Behältnis mit 100 mg rhBMP-2 versetzt und vorsichtig homogen verrührt. Anschließend wird ein boviner Spongiosa-Knochen mit den Ausmaßen 3×3×2 cm³ in die Kollagen/BMP-Lösung eingetaucht und unter Vakuum 10 Minuten in der Lösung belassen. Der so behandelte Spongiosa-Block wird dem Behältnis anschließend entnommen, in eine Wanne gegeben, in einer Gefriertrocknungsanlage stufenweise eingefroren und getrocknet. Schließlich wird der mit BMP angereicherte und mit Kollagen ummantelte Spongiosa-Block in geeigneter Weise sterilisiert.

Der nach dem beschriebenen Verfahren hergestellte Spongiosa-Block enthält in Kollagen eingeschlossenes BNIP, so daß eine vorzeitige Ausschwemmung bei einer chirurgischen Anwendung als Transplantat verhindert wird. Es erfolgt eine verzögerte Freisetzung von BMP nach Maßgabe des Kollagenabbaus.

Durch die induktive Wirkung der im Transplantat angereicherten BMP's erfolgt eine beschleunigte knochenneubildung an einer Defektstelle.

### Beispiel 2

100 ml einer in bekannter Weise hergestellten 1,0%igen Kollagenlösung werden in einem Behältnis mit 100 mg rhBMP-2 versetzt und vorsichtig homogen verrührt. Anschließend wird die BMP/Kollagenlösung in eine Metallwanne gegeben und es wird Knochenmaterial beispielsweise in Form von Chips aus bovinem Knochen von ungefähr 8 Millimetern Durchmesser hinzugefügt und zur besseren Durchdringung der Chips mit Lösung ein Vakuum angelegt. Die Wanne wird mit Inhalt in einer Gefriertrocknungsanlage stufenweise eingefroren und getrocknet. Schließlich wird das resultierende Produkt in geeigneter Weise sterilisiert.

## Patentansprüche

1. Verfahren zur Herstellung eines mit Knochenwachstumsfaktoren angereicherten Knochenmaterials, wobei die Oberfläche und/ oder das Innere des Knochenmaterials mit zumindest einem Knochenwachstumsfaktor (BMP) beladen und mit einem resorbierbaren Stoff ummantelt wird, wobei das Knochenmaterial zunächst durch Auf sprühen, Aufträufeln oder Einsaugen einer oder durch Eintauchen in eine Lösung oder Suspension des Knochenwachstumsfaktors beladen wird, und das so behandelte Knochenmaterial anschließend ummantelt wird.

2. Verfahren nach Anspruch 1,
**dadurch gekennzeichnet, daß** durch Anlegen eines Vakuums beim Aufsprühen, Aufträufeln oder Einsaugen der Lösung oder Suspension auch das Innere des Knochenmaterials mit Knochenwachstumsfaktoren beladen wird.

3. Verfahren nach Anspruch 1 oder 2,
**dadurch gekennzeichnet, daß** das beladene Knochenmaterial zur Ummantelung in vorgefertigte Kapseln, Mulden oder Taschen aus einem resorbierbaren Stoff eingebettet wird.

4. Verfahren nach Anspruch 1 oder 2,
**dadurch gekennzeichnet, daß** die Ummantelung der Oberfläche und/oder des Inneren des Knochenmaterials durch Aufsprühen, Aufträufeln oder Einsaugen einer Lösung oder Suspension des resorbierbaren Stoffes, vorzugsweise unter Vakuum, erfolgt.

5. Verfahren nach einem der vorhergehenden Ansprüche,
**dadurch gekennzeichnet, daß** als resorbierbarer Stoff Kollagen in Form einer Lösung, einer Suspension als Gel oder Gelatine, Polyglycolsäure, Polymilchsäure, Oxycellulose oder Gewebekleber, beispielsweise Fibrin- oder Acrylkleber, verwendet wird.

6. Verfahren nach einem der vorhergehenden Ansprüche,
**dadurch gekennzeichnet, daß** Knochenmaterial in Form von Blöcken, Chips oder Pulver verwendet wird.

7. Verfahren nach einem der vorhergehenden Ansprüche,
**dadurch gekennzeichnet, daß** ganz oder teilweise demineralisiertes Knochenmaterial verwendet wird.

8. Verfahren nach einem der vorhergehenden Ansprüche,
**dadurch gekennzeichnet, daß** das Knochenmaterial mit zumindest einem Antibiotikum beladen wird.

9. Verfahren nach einem der vorhergehenden Ansprüche,
**dadurch gekennzeichnet, daß** das beladene Knochenmaterial gefriergetrocknet wird.

10. Knochenmaterial, hergestellt durch ein Verfahren nach zumindest einem der vorstehenden Ansprüche.

11. Knochenmaterial nach Anspruch 10 zur Verwendung als chirugisches Transplantat.

## Claims

1. A method of manufacturing a bone material enriched with bone growth factors, wherein the surface and/or the interior of the bone material is charged with at least one bone growth factor (BMP) and is sheathed with a resorbable material, wherein the bone material is initially charged by spraying on, trickling on or sucking in of a solution or suspension of the bone growth factor or by immersion into a solution or suspension of the bone growth factor and the bone material treated in this manner is subsequently sheathed.

2. A method in accordance with claim 1, **characterized in that** the interior of the bone material is also charged with bone growth factors by application of a vacuum during the spraying on, trickling on or sucking in of the solution or suspension.

3. A method in accordance with claim 1 or claim 2, **characterized in that** the charged bone material is embedded in pre-fabricated capsules, depressions or pockets of a resorbable material for the sheathing.

4. A method in accordance with claim 1 or claim 2, **characterized in that** the sheathing of the surface and/or of the interior of the bone material takes place by spraying on, trickling on or sucking in of a solution or suspension of the resorbable material, preferably under vacuum.

5. A method in accordance with any one of the preceding claims, **characterized in that** collagen is used as the resorbable material in the form of a solution, of a suspension, as a gel or gelatin, polyglycol acid, polylactic acid, oxycellulose or fabric adhesive, for example fibrin adhesive or acrylic adhesive.

6. A method in accordance with any one of the preceding claims, **characterized in that** bone material is used in the form of blocks, chips or powder.

7. A method in accordance with any one of the preceding claims, **characterized in that** fully or partly demineralized bone material is used.

8. A method in accordance with any one of the preceding claims, **characterized in that** the bone material is charged with at least one antibiotic.

9. A method in accordance with any one of the preceding claims, **characterized in that** the charged bone material is freeze dried.

10. A bone material manufactured by a method in accordance with at least one of the preceding claims.

11. Use of a bone material in accordance with claim 10 as a surgical transplant.

## Revendications

1. Procédé de fabrication d'une substance osseuse enrichie en facteurs de croissance osseuse, dans lequel la surface et/ou l'intérieur de la substance osseuse est chargée d'au moins un facteur de croissance osseuse (BMP) et enrobée d'une matière résorbable, la substance osseuse étant dans un premier temps chargée par pulvérisation, instillation ou aspiration d'une solution ou suspension du facteur de croissance osseuse, ou par immersion dans cette dernière, et la substance osseuse ainsi traitée étant ensuite enrobée.

2. Procédé selon la revendication 1,
**caractérisé en ce que**, par l'application d'un vide lors de la pulvérisation, de l'instillation ou de l'aspiration de la solution ou suspension, l'intérieur de la substance osseuse est également chargé de facteurs de croissance osseuse.

3. Procédé selon la revendication 1 ou 2,
**caractérisé en ce que**, pour l'enrobage, la substance osseuse chargée est incorporée dans des capsules, cuvettes ou poches préfabriquées en une matière résorbable.

4. Procédé selon la revendication 1 ou 2,
**caractérisé en ce que** l'enrobage de la surface et/ou de l'intérieur de la substance osseuse par pulvérisation, instillation ou aspiration d'une solution ou suspension de la matière résorbable, est de préférence effectué sous vide.

5. Procédé selon l'une des revendications précédentes,
**caractérisé en ce que**, en tant que matière résorbable, il est utilisé du collagène à l'état de solution, de suspension en tant que gel ou gélatine, de l'acide polyglycolique, de l'acide polylactique, de l'oxycellulose ou de la colle pour tissu, par exemple de la colle acrylique ou à base de fibrine.

6. Procédé selon l'une des revendications précédentes,
**caractérisé en ce que** la substance osseuse est utilisée sous la forme de blocs, d'écailles ou de poudre.

7. Procédé selon l'une revendications précédentes,
**caractérisé en ce que** de la substance osseuse entièrement ou partiellement déminéralisée est utilisée.

8. Procédé selon l'une des revendications précédentes,
**caractérisé en ce que** la substance osseuse est chargée d'au moins un antibiotique.

9. Procédé selon l'une des revendications précédentes,
**caractérisé en ce que** la substance osseuse chargée est lyophilisée.

10. Substance osseuse fabriquée par un procédé selon l'une au moins des revendications précédentes.

11. Substance osseuse selon la revendication 10, destinée à être utilisée en tant que transplant chirurgical.
